(19) Europäisches Patentamt
European Patent Office
Office européen des brevets

(11) Veröffentlichungsnummer: **0 240 853**
**A2**

# (12) EUROPÄISCHE PATENTANMELDUNG

(21) Anmeldenummer: **87104464.0**

(51) Int. Cl.[4]: **A61K 31/53**

(22) Anmeldetag: **26.03.87**

(30) Priorität: **05.04.86 DE 3611425**

(43) Veröffentlichungstag der Anmeldung:
**14.10.87 Patentblatt 87/42**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Kreutzberger, Alfred, Prof. Dr.**
**Wormser Strasse 171f**
**D-6500 Mainz(DE)**
Erfinder: **Söder, Alfons, Dr.**
**Kauber Weg 1**
**D-6000 Frankfurt am Main(DE)**
Erfinder: **Gerhards, Hermann Josef, Dr.**
**Wacholderweg 4**
**D-6238 Hofheim am Taunus(DE)**

## (54) 2,4,6-Tris-tertiärbutylamino-1,3,5-triazin zur Prophylaxe und Behandlung von Epilepsie und Angstzuständen, Verwendung dieser Verbindung zur Herstellung eines Arzneimittels sowie Arzneimittel auf Basis dieser Verbindung.

(57) 2,4,6-Tris-tertiärbutylamino-1,3,5-triazin der Formel I

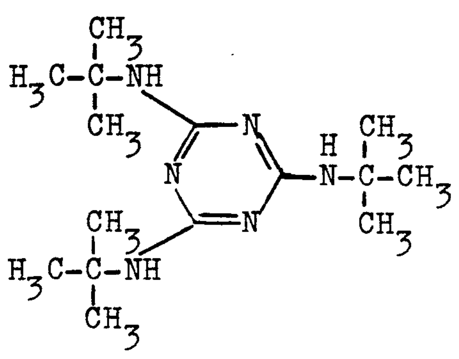

I

zur Prophylaxe und Behandlung von Epilepsie und Angstzuständen sowie die Verwendung zur Herstellung eines Arzneimittels zur Prophylaxe und Behandlung von Epilepsie und Angstzuständen werden beschrieben.

EP 0 240 853 A2

**2,4,6-Tris-tertiärbutylamino-1,3,5-triazin zur Prophylaxe und Behandlung von Epilepsie und Angstzuständen, Verwendung dieser Verbindung zur Herstellung eines Arzneimittels sowie Arzneimittel auf Basis dieser Verbindung**

Die Erfindung betrifft 2,4,6-Tris-tertiärbutylamino-1,3,5-triazin der Formel I

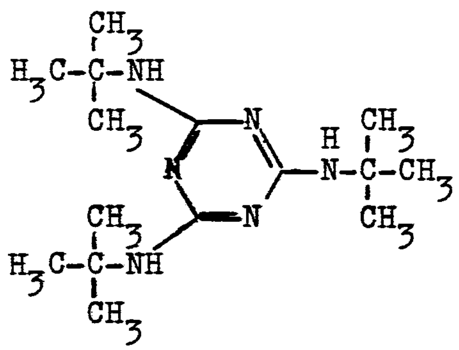

I

zur Prophylaxe und Behandlung von Epilepsie und Angstzuständen.

Das 2,4,6-Tris-tertiärbutylamino-1,3,5-triazin wurde bereits beschrieben (vergl. z.B. US-Patent 2.691.021). Entsprechend den Angaben in der Literatur findet dieses 1,3,5-Triazinderivat Verwendung bei der Herstellung von synthetischen Harzen und oberflächenaktiven Stoffen (US-Patent 2.691.021). Die Verwendung gegen Mycobakterien (US-Patent 3.591.693 und US-Patent 3.706.741) und gegen Fortschreiten von Arthritis (US-Patent 4.269.832) wurde ebenfalls beschrieben.

Es wurde nun überraschenderweise gefunden, daß 2,4,6-Tris-tertiärbutylamino-1,3,5-triazin zur Behandlung und Prophylaxe von epileptischen Erkrankungen und von Angstzusänden verwendet werden kann.

Die Erfindung betrifft daher 2,4,6-Tris-tertiärbutylamino-1,3,5-triazin zur Prophylaxe und Behandlung von Epilepsie und Angstzuständen.

Die Erfindung betrifft ferner Arzneimittel mit psychotroper Wirkung, welche eine Verbindung der Formel I enthalten, sowie die Verwendung dieser Verbindung zur Herstellung eines Arzneimittels. Schließlich betrifft die Erfindung auch ein Verfahren zur Prophylaxe und Behandlung von epileptischen Erkrankungen und Angstzuständen.

Die Herstellung von 2,4,6-Tris-tertiärbutylamino-1,3,5-triazin erfolgt wie in der Literatur beschrieben z.B. durch basen-oder säurekatalysierte Trimerisation von tertiär-Butylcyanamid (US-PS 2.691.021), durch Umsetzung von 2,4-Bis(tert.butylamino)-6-chlor-1,3,5-triazin mit tertiär Butylamin (US-PS 3.591.693) oder durch Umsetzung von Cyanurchlorid mit einem Überschuß von tertiär Butylamin (US-PS 4.296.832).

Das Triazinderivat 2,4,6-Tris-tertiärbutylamino-1,3,5-triazin besitzt pharmakologische Eigenschaften, die es als mögliches Krampf-verhinderndes und Angst-lösendes Pharmakon charakterisiert.
Die Krampf-verhindernde (antikonvulsive) Wirkung der erfindungsgemäßen Verbindung wurde an Mäusen gegen Pentetrazol-und gegen Elektroschock-induzierte Krämpfe in der Anordnung E.A. Swinyard geprüft.

1. Antagonismus gegen Pentetrazol-induzierte Krämpfe (Maus)

Methode (E.A. SWINYARD, J. Pharmacol. Exp. Ther. 106, (1952), 319-330):

Gruppen von 10 männlichen Mäusen (Stamm: NMRI; SPF-71, KF) im Gewicht von 18-22 g werden in diesem Versuch verwendet. Die zu prüfenden Substanzen werden in einer 1 %igen Aufschwemmung von ®Tylose (Methyl-hydroxy-ethyl-cellulose) in Wasser suspendiert und per Schlundsonde in einem Volumen von 10 ml/kg Körpergewicht oral verabreicht. Die Kontrollgruppe erhält ein entsprechendes Volumen Tylosesuspension ohne Prüfsubstanz. Pentetrazol (Cardiazol (R)) wird in wässriger Lösung in einer Dosis von 125 mg/kg Körpergewicht 1 Stunde nach Verabreichung der Prüfsubstanzen subcutan injiziert. Diese Dosis erzeugt bei 90-100 % der Kontrolltiere innerhalb der Beobachtungszeit von 60 Minuten nach der Injektion tonische Extensorkrämpfe der hinteren Extremitäten. Als Schutzwirkung der Prüfsubstanz wird gewertet, wenn die Anzahl der krampfenden Tiere gegenüber der Kontrollgruppe vermindert ist.

Bei vorhandener Schutzwirkung der Prüfsubstanz wird eine mittlere effektive Dosis (ED-50) grafisch (Lichtfield and Wilcoxon) oder rechnerisch (Probitanalyse) ermittelt.

Die ermittelte $ED_{50}$ (50 %-Hemmdosis) beträgt nach oraler Verabreichung 3,6 mg/kg Versuchstier.

2. Antagonismus gegen Elektroschock-induzierte Krämpfe (Maus)

Methode (E.A. SWINYARD, In: Experimental Models of Epilepsy, Raven Press, New YORK, 1972, p. 433-458):

Gruppen von 6 männlichen Mäusen (Stamm: NMRI, SPF-71, KF) im Gewicht von 18-22 g werden in diesem Versuch verwendet. Die zu prüfenden Substanzen werden in einer 1 %igen Aufschwemmung von Tylose (Methyl-hydroxy-ethyl-cellulose) in Wasser suspendiert und per Schlundsonde in einem Volumen von 10 ml/kg Körpergewicht oral verabreicht. Die Kontrollgruppe erhält ein entsprechendes Volumen Tylosesuspension ohne Prüfsubstanz.

Eine Stunde nach Prüfsubstanzgabe wird den Tieren über Cornea-Elektroden ein Elektroshock verabreicht (Stromstärke 12-22 mA, Dauer 0.2 sec, 50 Hz) nachdem vorher an einer zweiten Kontrollgruppe die Stromstärke ermittelt worden war, die bei 100 % der Tiere einen tonischen Extensorkrampf der hinteren Extremitäten hervorruft.

Die Anzahl der durch die Prüfsubstanz vor einem Extensorkrampf geschützten Tiere dient als Maß der antikonvulsiven Wirkung in diesem Test.

Bei vorhandener Schutzwirkung der Prüfsubstanz wird eine mittlere effektive Dosis (ED-50) grafisch (Litchfield and Wilcoxon) oder rechnerisch (Probitanalyse) ermittelt.

Die ermittelte $ED_{50}$ (50 %-Hemmdosis) beträgt nach oraler Verabreichung etwa 30 mg/kg Versuchstier.

Die Angst-lösende (anxiolytische) Wirkung der erfindungsgemäßen Verbindung wurde im "Lick-shock-conflict-Test" nach J.R. Vogel (Psychopharmacologia 21 (1971) 1-7) und im "Geller-Seifter Konflikt-Test" nach I. Geller und J. Seifter (Psychopharmacologia 1 (1962), 482-492) an WISTAR-RATTEN geprüft.

3. "Lick-stock-conflict" Test:

Methode:

Männliche Wistar Ratten aus eigener Aufzucht (SPF Hatterscheim) im Gewicht zwischen 90 und 120 g werden verwendet. Den Tieren wird für 48 Stunden vor Testbeginn das Trinkwasser entzogen. Zum Test werden die Tiere in eine Plastikbox (14x12x28 cm, BxTxH) gesetzt, die mit einer Wasserflasche mit Metall-Trinkrohr ausgestattet ist und die außerdem erlaubt, über eine elektronische Schaltung die Anzahl der Kontakte der Zunge des Tieres mit dem Trinkrohr zu messen. Der Boden der Box besteht aus Metallstäben, die durch die Steuerungselektronik unter Strom gesetzt werden können.

Die Tiere haben nach dem Einsetzen in die Box 5 min. Zeit, um das Trinkrohr zu finden und 50-mal daran zu lecken. Tiere, die das Trinkrohr innerhalb diese Zeit nicht gefunden haben, werden für den Versuch nicht verwendet. Nach diesen 50 Leckvorgängen (lickings) werden Trinkrohr und Bodenstäbe für jeweils 5 sec. unter Strom gesetzt (Gleichstrom, 300 µA) und dann für weitere 5 sec. wieder freigegeben. Diese Folge wird alternierend für eine Zeit von 5 min. weitergeführt, wobei die Zahl der Trinkrohr-Kontakte des Tieres während der Reizstrom-und der reizstromfreien Phase auf verschiedenen elektronischen Zählern registriert werden.

Gruppen von jeweils acht Tieren pro Dosis werden mit der Prüfsubstanz, die in einem 1 %igen Tylose-Gel aufgeschwemmt ist, oral per Schlundsonde behandelt. Das Injektionsvolumen beträgt 5 ml/kg Körpergewicht. Die Prüfung in der obg. Testapparatur erfolgt 1 und 2 Std. nach Applikation der Prüfsubstanz. Die Anzahl der Trinkrohr-Kontakte in der Reizstrom-Phase dient als Prüf-Variable. Die mittlere Anzahl der Kontakte in dieser Phase bei der Kontrollgruppe wird zu 100 % gesetzt und Zu-oder Abnahme der Kontaktzahl bei den mit Prüfsubstanz behandelten Tieren wird prozentual in Bezug auf die Kontrollgruppe ausgedrückt.

Anxiolytika bewirken in diesem Test gewöhnlich eine deutliche Zunahme der Wasseraufnahme (lickings) in der Reizstromphase gegenüber den unbehandelten Kontrollen. Falls eine lineare oder logarithmische Beziehung zwischen Dosis und Wirkung gegeben ist, wird eine ED +100 (d.i. die Dosis, die eine Zunahme der Wasseraufnahme um 100 % gegenüber der Kontrollgruppe bewirkt) mittels Regressionsanalyse errechnet. Ist eine lineare Dosisabhängigkeit nicht gegeben, so wird eine minimal effektive Dosis (MED) bestimmt, d.i. die niedrigste Dosis der Prüfsubstanz, die noch eine statistisch signifikante Zunahme der Wasseraufnahme im Vergleich zur Kontrollgruppe bewirkt (P = 0.05, DUNNET-Test).

Die ermittelte "minimal effective "Dosis (MED) nach oraler Gabe liegt zwischen 2 und 10 mg/kg Versuchstier, die prozentuale Zunahme der Wasseraufnahmen (lickings) beträgt 90 % (Dosis: 3 mg) bzw. 125 % (Dosis: 10 mg).

4. Geller-Seifter-Konflikt-Test

Methode:

Männliche Wistar Ratten aus eigener Aufzucht (SPF Hattersheim) im Gewicht zwischen 240 und 370 g werden verwendet und Versuchsgruppen zu je 8 Tieren zugeordnet. Je 4 Tiere werden in Plastikkäfigen (56x38x20 cm) zusammengesetzt und durch abgewogene Futtermengen auf ca. 80 % ihres normalen Körpergewichts gehalten.

Die Tiere werden trainiert, in einer SKINNER-Box eine Taste zu drücken, um eine Belohnung in Form von gesüßter Kondensmilch zu erhalten. Die Box enthält zwei Tasten mit Mikroschaltern, einen Lautsprecher, ein Hauslicht, zwei Signallichter über den Tasten sowie einen Fußboden aus Metallstäben. Das Trainingsschema wurde der Arbeit von GELLER und SEIFTER (1962) in der Modifikation von DAVIDSON & COOK (Psychopharmacologia 15 (1969), 159-168) entlehnt:
Jede Sitzung besteht aus vier 15-Minuten-Abschnitten, die alle aus einer 12-minütigen "Variable Interval" (VI) Phase und einer 3-minütigen "Fixed ratio" (FR) Phase zusammengesetzt sind. Währen der VI - Phase erhalten die Tiere auf Tastendruck Milchbelohnungen in einem durch einen Zufallsgenerator gesteuerten Intervall von 10-110 sec. mit einem Mittelwert um 60 sec. Während der FR-Phase erhalten die Tiere für jeden Tastendruck eine Belohnung, zusätzlich wird jedoch bei jedem 3. Tastendruck ein schmerzhafter elektrischer Reiz über die Fussbodenstäbe verabreicht, um eine Konfliktsituation zu erzeugen.
Die Stromstärke des elektrischen Reizes wird für jedes Tier individuell so eingestellt (0,3-0,6 mA), daß die Tastendruckrate in der gesamten FR-Phase zwischen 5 und 15 liegt.

Das Training findet an 5 Tagen in der Woche statt, Versuche mit Prüfsubstanzen werden einmal wöchentlich durchgeführt. Da die Tiere als ihre eigene Kontrolle dienen, werden vor jedem Versuch mit der Prüfsubstanz mindestens zwei Vorwerte ohne Prüfsubstanz durchgeführt. Die zu prüfende Verbindung wird in einem 1 %igen Tylose-Gel suspendiert und 30 min vor Testbeginn oral per Schlundsonde in einem Volumen von 2 ml/kg verabreicht. Veränderungen der Tastendruckrate in der VI-Phase werden als Beeinflussung der motorischen Aktivität und Steigerung der Tastendruckrate in der FR-Phase werden als Anzeichen für eine "Antikonflikt"-bzw. "anxiolytische Wirkung" gewertet. Bestimmt wird meist die minimal effektive Dosis (MED) der Prüfsubstanz, d.i. die niedrigste geprüfte Dosis, die noch eine statistisch signifikante Änderung der Tastendruckrate bewirkt (p = 0,05; WILCOXON matched pairs signed rank test).

Die MED nach oraler Gabe liegt zwischen 3 und 10 mg/kg Versuchstier. Die prozentuale Zunahme der angenommenen Belohnungen beträgt 158 % (Dosis 3 mg) bzw. 235 % (Dosis 10 mg).

Da epiliptische Anfälle häufig durch Streß und Angstsituationen ausgelöst werden, ist die Kombination von antiepileptischer und anxiolytischer Wirkung der erfindungsgemäßen Verbindung besonders wertvoll.

Aufgrund der aufgezeigten Wirkungen eignet sich die Verbindung der Formel I zur Prophylaxe und Behandlung von epileptischen Erkrankungen und Angstzuständen.

Die erfindungsgemäßen Arzneimittel zur Prophylaxe und Behandlung von Epilepsie und Angstzuständen enthalten das Triazinderivat der Formel I als Wirkstoff, gegebenenfalls in Kombination mit anderen Wirkstoffen.

Die Arzneimittel werden nach an sich bekannten, dem Fachmann geläufigen Verfahren hergestellt. Als Arzneimittel wird eine Wirksame Menge des genannten Wirkstoffs entweder per se oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorzugsweise zwischen 10 und 75 % beträgt. Welche Hilfsstoffe für die gewünschte Arzneimittelformulierung geeignet sind, ist dem Fachmann aufgrund seines Fachwissens geläufig. Neben Tablettenhilfsstoffen, Lösemitteln, Gelbildnern, Suppositoriengrundlagen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Der Wirkstoff kann oral, parenteral, intravenös oder rektal appliziert werden, wobei die orale Applikation bevorzugt ist. Für eine orale Anwendungsform wird der genannte Wirkstoff gegebenenfalls mit weiteren aktiven Verbindungen mit den dafür geeigneten Zusatzstoffen, wie Trägerstoffen, Stabilisatoren oder inerten Verdünnungsmitteln, vermischt und durch die üblichen Methoden in geeignete Darreichungsformen gebracht, wie Tabletten, Dragees, Steckkapseln, sowie wäßrige, alkoholische oder ölige Suspensionen beziehungsweise Lösungen. Als inerte Träger können z.B. Gummi arabicum, Magnesia, Milchzucker, Glucose oder Stärke, insbesondere Maisstärke, verwendet werden. Dabei kann die Zubereitung sowohl als Trocken-als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation wird der Wirkstoff gewünschtenfalls mit den dafür üblichen Substanzen wie Lösungsvermittler, Emulgatoren oder weiteren Hilfsstoffen in Lösung, Suspension oder Emulsion gebracht. Als Lösungmittel kommen zum Beispiel in Frage Wasser, physiologische Kochsalzlösung oder Alkohole, zum Beispiel Ethanol, Propanol, Glycerin, daneben auch Zuckerlösungen wie Glucose-ode Mannitlösungen, oder auch eine Mischung aus den veschiedenen genannten Lösungsmitteln.

Als täglich zu verabreichende Dosis ist eine solche zu wählen, die dem gewünschten Effekt angepaßt ist. Pro Patient werden täglich etwa 5 bis 200 mg, vorzugsweise oral, verabreicht. Bei der Anwendung als Antiepileptikum beträgt die Dosierung vorzugsweise 10 bis 100 mg täglich, wobei die Dosierung je Einheit zweckmäßigerweise 2,5 bis 50 mg vorzugsweise 2,5 bis 25,0 mg beträgt. Als Anxiolytikum werden täglich etwa 5 bis 50 mg in Einzeldosierungen zwischen etwa 1,0 und 20 mg verabreicht. Es können selbstverständlich auch darüber oder darunter liegende Dosierungseinheiten verwendet werden, die gegebenenfalls vor Applikation zuteilen bzw. zu vervielfachen sind.

Beispiel 1

Dragees

Für die Herstellung von 1000 Dragees werden 25 g 2,4,6-Tris-tertiärbutylamino-1,3,5-triazin, 50 g Maisstärke, 35 g Laktose, 8,5 g Talkum, 1 g Magnesiumstearat und 0,5 g kolloidale Kieselsäure gemischt und zu Drageekernen von 120 mg Gewicht verpreßt. Die Kerne werden mit einem aus Dragiergemisch aus 44,57 g Rohrzucker, 23,4 g Talkum, 8 g Celluloseacetatphthalat, 2,24 g Ricinusöl sowie geringe Zusätze von Gummi Arabicum, Titandioxyd und Wachs, das so eingesetzt wird, daß das Endgewicht der Dragees 200 mg beträgt, überzogen.

Beispiel 2

Tabletten

1000 Tabletten werden aus folgender Mischung hergestellt: 50 g 2,4,6-Tris-tertiärbutylamino-1,3,5-triazin, 35 g Laktose, 50 g Maisstärke, 13 g Talkum und 2 g Magnesiumstearat. Wirk-und Hilfsstoffe werden gemischt und gegebenenfalls nach vorheriger Granulation, zu Tabletten verpreßt.

Beispiel 3

Kapseln

Für die Herstellung von 1000 Steckkapsen mischt man 10 g 2,4,6-Tris-tertiärbutylamino-1,3,5-triazin, 46 g Maisstär ke, 40 g Laktose und 4 g kolloidale Kiselsäure. Füllgewicht 100 mg/Steckkapsel.

Beispiel 4

Orale Suspension

Die aus 50 g Sorbit, 300 mg Natriumsalz der Benzosäure, 20 mg Saccharin, 100 mg Fruchtaroma sowie 1 g 2,4,6-Tris-tertiärbutylamino-1,3,5-triazin und aqua dest. ad 100 ml gewonnene orale Suspension enthält 10 mg des Wirkstoffes pro ml.

## Ansprüche

1. 2,4,6-Tris-tertiärbutylamino-1,3,5-triazin der Formel

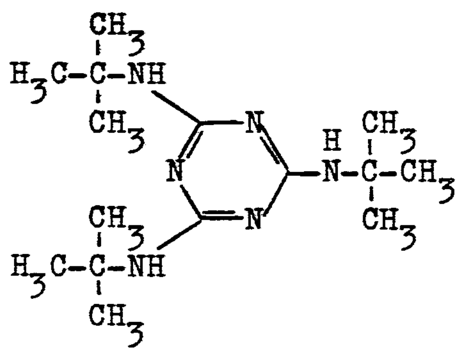

I

zur Prophylaxe und Behandlung von Epilepsie und Angstzuständen.

2. Arzneimittel zur Prophylaxe und Behandlung von Epilepsie und Angstzuständen, dadurch gekennzeichnet, daß es eine wirksame Menge an 2,4,6-Tris-tertiärbutylamino-1,3,5-triazin und pharmakologisch verträgliche Trägerstoffe enthält.

3. Verfahren zu Herstellung eines Arzneimittel zur Prophylaxe und Behandlung von Epilipsie und Angstzuständen gemäß Anspruch 2, dadurch gekennzeichnet, daß man 2,4,6-Tris-tertiärbutylamino-1,3,5-triazin mit einem pharmakologisch akzeptablen, pharmazeutischen Trägerstoff in eine geeignete Darreichungsform überführt.

4. Verwendung von 2,4,6-Tris-tertiärbutylamino-1,3,5-triazin zur Behandlung und Prophylaxe von Epilepsie und Angstzuständen.

5. Verwendung von 2,4,6-Tris-tertiärbutylamino-1,3,5-triazin zur Herstellung eines Arzneimittels zur Behandlung und Prophylaxe von Epilepsie und Angstzuständen.

6. Verfahren zur Behandlung und Prophylaxe von Epilepsie und Angstzuständen, dadurch gekennzeichnet, daß man eine wirksame Menge der Verbindung gemäß Anspruch 1 verabreicht.

Patentansprüche für die folgenden Vertragsstaaten: Griechenland, Österreich und Spanien:

1. Verfahren zur Herstellung eines Arzneimittels zur Prophylaxe und Behandlung von Epilepsie und Angstzuständen, dadurch gekennzeichnet, daß man eine wirksame Menge an 2,4,6-Tris-tertiärbutylamino-1,3,5-triazin der Formel I

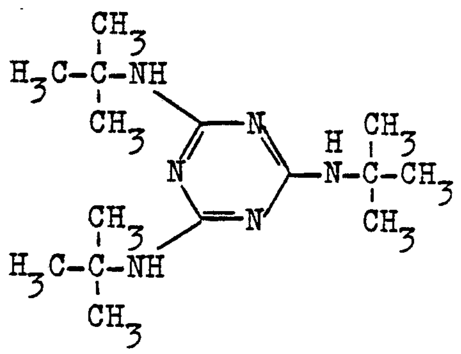

I

mit einem pharmakologisch akzeptablen, pharmazeutischen Trägerstoff in eine geeignete Darreichungsform überführt.

2. Verwendung von 2,4,6-Tris-tertiärbutylamino-1,3,5-triazin zur Behandlung und Prophylaxe von Epilepsie und Angstzuständen.

3. Verwendung von 2,4,6-Tris-tertiärbutylamino-1,3,5-triazin zur Herstellung eines Arzneimittels zur Behandlung und Prophylaxe von Epilepsie und Angstzuständen.